# EUROPEAN PATENT SPECIFICATION

(11) **EP 1 198 225 B1**
(45) Date of publication and mention of the grant of the patent: **11.01.2006**
(21) Application number: 00949439.4
(22) Date of filing: 02.08.2000
(51) Int. Cl.: A61K 9/127, A61K 31/425

(54) **EPOTHILONE COMPOSITIONS**
EPOTHILON ZUSAMMENSETZUNGEN
COMPOSITIONS D'EPOTHILONE

(30) Priority: 04.08.1999 GB 9918429
(43) Date of publication of application: 24.04.2002
(73) Proprietor: Novartis AG, 4056 Basel (CH); Novartis Pharma GmbH, 1230 Wien (AT)
(72) Inventor: SONNTAG, Jean-Claude, F-68260 Kingersheim (FR); WARTMANN, Markus, CH-4125 Riehen (CH); ALTMANN, Karl-Heinz, CH-4153 Reinach (CH)
(74) Representative: de Weerd, Petrus G.W.
(86) International application number: PCT/EP2000/007488
(87) International publication number: WO 2001/010412

(56) References cited:
- WO-A-98/24427

## Description

This invention relates to liposomal compositions, e.g. in solid form or in form of a suspension, comprising an epothilone, suitable for inhalation, topical, ophthalmic, intraarticular, intrathecal, oral and parenteral, e.g. intravenous, administration.

The epothilones represent a class of microtubule stabilizing cytotoxic agents (see Gerth, K. et al., J. Antibiot. 49, 560-3 (1966); or Hoefle et al., DE 41 38 042) of the formula I. Typical representatives include epothilone A wherein R is a hydrogen and epothilone B wherein R is a methyl group.

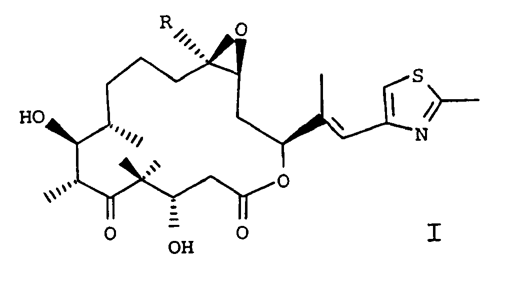

They are 16-member macrolides containing seven chiral centers and may also be characterized by various functionalities. For example, they may include other ring systems, such as an epoxide and/or a thiazole ring. They may have two free, derivatizable hydroxyl groups and the macrolide itself may comprise an ester linkage. The epothilones and their syntheses are described for example in published PCT application number WO 93/10121 and DE 41 38 042 A2. Typical epothilone derivatives and their syntheses are described in published PCT application number WO 99/27890, WO 99/07692, WO 99/02514, WO 99/01124, WO 97/19086 and WO 98/25929.

The term "epothilones" as used herein includes epothilone A or epothilone B, analogues, e.g. derivatives, or mixtures thereof as appropriate. Epothilone A or B may be used alone or they may be used as mixtures of A and B. Preferably, however, they are used as solely A or solely B, most preferably solely B.

Cytotoxic agents are well known for the treatment of tumours. The anti-tumour activity of many of these compounds relies on the inhibition of cell proliferation and consequent induction of apoptosis and cell death. The majority of cytotoxic agents exert their effects through interference of DNA and/or RNA syntheses. However, for certain cytotoxic agents, e.g. members of the taxane family, e.g. paclitaxel, and the epothilones, their activity is reliant on their interference with microtubule dynamics. Microtubules are an important and attractive target for development of novel anti-cancer compositions.

However, little has been published on compositions suitable for epothilones. Yet, the therapeutic benefits of epothilones when given systemically may be accompanied by unfavorubale side effects due to their toxicity. In addition, we have found that the 16-member macrolide system is particularly labile to degradation which further may impair therapeutic efficacy of an epothilone.

The present applicants have now surprisingly found means to decrease the unfavourable side effects while simultaneously improving the therapeutic efficacy of an epothilone.

Accordingly, this invention provides in one of its aspects a liposomal composition comprising an epothilone, e.g. an epothilone-liposome.

In another aspect this invention provides a pharmaceutical composition comprising an epothilone-liposome.

The above liposomal and pharmaceutical compositions may hereinafter also be referred to as compositions of the present invention.

Suitable epothilone-liposomes are based on various lipid components and conveniently include those having lipids which form vesicles, preferably vesicle-forming lipids having two hydrocarbon chains, typically acyl chains, and a polar head group. Typical lipids embrace for example
(i) phopsholipids, which include
   (a) phosphatidylcholine (PC), e.g. dimyristoyl phospatidylcholine, dipalmitoyl phosphatidylcholine, distearoyl phosphatidylcholine, dioleyl phosphatidylcholine, dilinoeloyl phosphatidylcholine, dilauryolyl phosphatidylcholine, and the like,
   (b) phosphatidylglycerols (PG), e.g. dilauryloyl phosphatidylglycerol, dimyristoyl phosphatidylglycerol, dipalmitoyl phosphatidylglycerol and dioleyl phosphatidylglycerol,
   (c) phosphatidic acids (PA), e.g. dimyristoyl phosphatidic acid and dipalmitoyl phosphatidic acid,
   (d) phosphatidylethanolamines (PE), e.g. distearyl phosphatidylethanolamine, dimyristoyl phosphatidylethanolamine and dipalmitoyl phosphatidylethanolamine,
   (e) phosphatidylserines (PS), e.g. dimyristoyl phosphatidylserine, dipalmitoyl phosphatidylserine and dioleyl phosphatidylserine,
   (f) phosphatidylinositols (PI), and
   (g) sphingomyelins (SM),
(ii) glycolipids, and
(iii) cholesterol.

Preferably the two hydrocarbon chains of the above-described phospholipids are preferably between about 14 to 22 carbon atoms in length. If desired they may have varying degrees of unsaturation.

In another aspect, the invention provides a composition comprising an epothilone-liposome containing at least one lipid, e.g. one or more phospholipids or a combination of one or more phospholipids with a glycolipid and/or cholesterol.

The above-described lipids whose acyl chains have a variety of degrees of saturation may be obtained commercially, or prepared according to published methods.

Preferred epothilone-liposomes may include a vesicle-forming lipid which contains a polymer chain. The vesicle-forming lipids which may be used are any of those described above for the first vesicle-forming lipid component, preferably a phospholipid, for example a phosphatidylethanolamine (PE), e.g. distearyl phosphatidylethanolamine (DSPE).

The polymer in the derivatized lipid may be any hydrophilic polymer including polyethyleneglycol, polyvinylpyrrolidone, polymethyloxazoline, polyethyloxazoline, polyhydroxypropyl methacrylamide, polymethacrylamide, polydimethylacrylamide, polylactic acid, polyglycolic acid, and derivatized celluloses, such as hydroxymethylcellulose or hydroxyethylcellulose. Preferably, the polymer is polyethyleneglycol (PEG), preferably as a PEG chain having a molecular weight between 500 to 10,000 daltons, more preferably between 500 and 5,000 daltons, most preferably between 500 and 2,000 daltons. Such lipids containing PEG-derivatized lipids have been referred to as Stealth liposomes.

Thus in another aspect, the invention provides a composition comprising an epothilone-liposome containing one or more polymer-derivatized lipids, preferably polymer-derivatized phospholipids, e.g. polyethyleneglycol-derivatized phospholipids.

Such derivatized vesicle-forming lipid may further be modified by coupling targeting moieties, e.g. polypeptides, e.g. antibody molecules to the liposome. For example, a hydrophilic polymer chain attached to the lipid may carry a functional end group, e.g. a hydrazide or hydrazine group or a (2-pyridyldithio) propionamide) group, to facilitate coupling of targeting moieties. The polymer in the derivatized lipid may be end-functionalized polyethyleneglycol, polyvinylpyrrolidone, polymethyloxazoline, polyethyloxazoline, polyhydroxypropyl methacrylamide, polymethacrylamide, polydimethylacrylamide, polylactic acid, polyglycolic acid, and derivatized celluloses, such as hydroxymethylcellulose or hydroxyethylcellulose, preferably end-functionalized polyethyleneglycol.

Preferably the weight ratio of lipid to an epothilone in an epothilone-liposome may range from 1:99 to 90:1, e.g. 50:1 to 10:1, is preferably high e.g. greater than 10:1. The ratio may depend on the nature and composition of the epothilone-liposome, but may be preferably as high as 20:1. The amount of polymer-derivatized lipid is preferably from 0.5 to 50% of the lipid content. The lipid components are preferably present in a molar ratio of about 30-75 % vesicle-forming lipids, 25-40 % cholesterol, and 1-20 % polymer-derivatized lipid.

An epothilone-liposome may be prepared using conventional methods to obtain drug-containing liposomes, for example by simple lipid-film hydration techniques. In this procedure, a mixture of liposome-forming lipids of the type detailed above dissolved in a suitable organic solvent is evaporated in a vessel to form a thin film, which is then covered by an aqueous medium. The lipid film hydrates to form vesicles, typically with sizes between about 0.1 to 10 microns. Alternatively, an epothilone-liposome may be prepared by vortexing the dried lipid film in a buffered aqueous solution. The epothilone-liposome may generally be prepared by adding an epothilone to the vesicle-forming lipids prior to liposome formation to entrap the epothilone in the formed liposome. For example, a solution of an epothilone and one or more lipids dissolved in an organic solvent may be added gradually to a stirred aqueous medium to give an aqueous suspension of epothilone-liposomes. The solvent may then be removed from the resulting suspension, for example by solvent evaporation or lyophilisation, or dialysis. In a further method, an epothilone may be incorporated into preformed liposomes by active transport mechanisms, such as remote loading, e.g. the epothilone is taken up in liposomes in response to a gradient, such as an ammonium sulphate gradient or a potassium or hydrogen ion concentration differential.

In another aspect, the invention provides a composition comprising an epothilone-liposome and a pharmaceutically acceptable solvent.

Epothilone-liposomes may have a particle size within a suitable range for the administration contemplated. Preferably the mean particle diameter is less than 10 µm (microns), preferably from about 0.5 to about 8 µm (microns), more preferably from about 0.5 to about 5 µm (microns), most preferebly from about 0.5 to about 4 µm (microns). The particle size may be reduced to the desired level by conventional methods, for example by using in an air-jet mill, ball mill or vibrator mill, microprecipitation, spray-drying, lyophilisation, high-pressure homogenisation, recrystallisation from supercritical media, or by extruding an aqueous suspension of epothilone-liposomes through a series of e.g. polycarbonate membranes having a selected uniform pore size to obtain epothilone-liposomes of desired size distribution.

Epothilone-liposomes may be dehydrated, for example by lyophilisation preferably in the presence of a cryoprotectant, to give epothilone-liposomes in form of a dry powder. Suitable cryoprotectants may include a sugar, for example a monosaccharide such as glucose, a polymeric sugar such as dextran or, preferably a disaccharide such as sucrose, lactose, maltose or trehalose.

Thus, in another aspect, the invention provides a composition comprising an epothilone-liposome in dry form.

Such epothilone-liposomes in dry form are conveniently stored in suitable containers, e.g. vials, double-chamber vial or syringe systems, syringes or ampoules. The container forms may be of any suitable size conventional in the art. By "suitable size" is meant an appropriate size having regard to the volume of solution which will be needed to reconstitute the dry composition. Any suitable containers may be used to provide these dosage forms. By "suitable" is meant any container which may be used in aseptic filling procedures and which is capable of maintaining a sterile environment and which is unreactive to the composition. Preferred containers may be formed of glass, e.g. borosilicate or soda-lime glass, or plastics materials, and may have means to receive a stopper, e.g. a sterile rubber stopper which may cooperate with the walls of the container to provide a hermitic seal. Preferred stoppers also may allow entry to the contents of the container for the purpose of introduction of a solvent, e.g. water for injection.

Epothilone-liposomes according to the invention may be surprisingly storage stable for an extended period of time, e.g. up to 24 to 36 months at a temperature of 2 to 30 °C and display no signs of degradation and the solubility characteristics remain unaffected.

Epothilone-liposomes in dry form may be re-constituted, preferably just before administration. Re-constitution may involve suspending the dry epothilone-liposomes in a pharmaceutically acceptable solvent, e.g. an aqueous, organic or aqueous/organic medium, chosen from media conveniently used to form liposomal suspensions compositions, such as buffers, for example acetate, ascorbate, phosphate, tartaric, citrate buffers and the like, before the epothilone is administered to a patient. Preferred buffers include citric acid, tartaric acid and sodium salts. These buffers may act themselves as antioxidants, or else other physiologically acceptable antioxidants may be added. Other components may be added, for example a physiologically acceptable surfactant or co-solvent to enhance solubility of the active agent and an isotonic agent or agents, e.g. a physiologically acceptable salt, to provide an isotonic composition.

Suitable physiologically acceptable surfactants used in compositions of the present invention may include for example sorbitan oleates, polyoxyethylenes, e.g. polyoxyethylene ethers, lecithins and the like. The isotonic agent or agents may be selected from any of those known in the art, e.g. mannitol, lactose, dextrose, glucose and sodium chloride. Preferably the isotonic agent is glucose, lactose or sodium chloride. The isotonic agent or agents may be used in amounts which impart to the infusion solution the same or essentially the same osmotic pressure as body fluid. The precise quantities needed can be determined by routine experimentation and may depend upon the composition of the pharmaceutical composition and the nature of the isotonic agent or agents. Selection of a particular isotonic agent or agents may be made having regard to the properties of the epothilone, e.g. epothilone A or epothilone B. For example, when epothilone B is employed alone or in combination with epothilone A, the use of certain isotonic agent or agents may cause the infusion solution to turn turbid. The turbidity may be attributed to precipitation of the epothilone, e.g. epothilone B.

The concentration of isotonic agent or agents in the aqueous medium will depend upon the nature of the particular isotonic agent or agents used and other components of the suspension. When glucose is used it is preferably used in a concentration of from 1 to 5% w/v, more particularly 5% w/v. When the isotonic agent is sodium chloride it is preferably employed in amounts of up to 1% w/v, in particular 0.9% w/v.

Thus, in yet another aspect, the invention provides a reconstituted form of a composition comprising an epothilone-liposome and a pharmaceutically acceptable solvent.

The compositions of the present invention may comprise other excipients, e.g. antioxidants. Antioxidants may be employed to protect the epothilone, e.g. epothilone B, against oxidative degradation. Antioxidants may be chosen from any of those antioxidants known in the art and suitable for the administration contemplated. The amount of antioxidant may be determined by routine experimentation. As an alternative to the addition of an antioxidant, or in addition thereto, the antioxidant effect may be achieved by displacing oxygen (air) from contact with the compositions of the invention. This may be conveniently carried out by purging the container holding said infusion solution with an inert gas, e.g. nitrogen.

The compositions of the present invention may be prepared using conventional techniques, for example sterile filtration and aliquots may then be filled into sterile containers, e.g. vials, syringes or ampoules.

The compositions of the present invention are useful for treatment and prevention of malignant proliferative disorders, for example the indications and conditions disclosed in WO 93/10121 and DE 41 38 042 A2, the contents of which are incorporated herein by reference. More specifically, they may be useful for the treatment of a proliferative disease, especially according to certain treatment regimens using an epothilone, especially epothilone B; preferably of a gastrointestinal tumor, more preferably
(1) a tumor of the colon and/or the rectum (colorectal tumor), especially if it is refractory to a (meaning at least one) representative of the taxane class of anti-cancer agents, in particular TAXOL® (paclitaxel in formulated form for clinical use), and/or at least one standard treatment with an other chemotherapeutic, especially 5-fluorouracil;
(2) a tumor of the genitourinary tract, more preferably a tumor of the prostate, including primary and metastatic tumors, especially if refractory to hormone treatment ("hormone refractory prostate cancer") and/ or treatment with other standard chemotherapeutics;
(3) an epidermoid tumor, more preferably an epidermoid head and neck tumor, most preferably a mouth tumor;
(4) a lung tumor, more preferably a non-small cell lung tumor, especially any of these tumors that is refractory to treatment with one or more other chemotherapeutics (especially due to multidrug resistance), especially to treatment with a member of the taxane class of anti-cancer agents, in particular TAXOL®; or
(5) a breast tumor, more preferably one that is multidrug resistant, especially refractory to treatment with a member of the taxane class of anti-cancer agents, in particular TAXOL®; relating especially also to the treatment of a multidrug resistant lung tumor (preferably a non-small cell lung tumor), a multidrug resistant breast tumor, or a multidrug resistant epidermoid tumor, or in a broader sense of the invention to a treatment schedule for the treatment of an aforementioned or (in a broader sense of the invention) any other tumor, especially if it is refractory to one or more chemotherapeutics, especially multidrug resistant and/or TAXOL® refractory), such as a melanoma, ovarian cancer, pancreas cancer, neuroblastoma, head and neck cancer or bladder cancer, or in a broader sense renal, brain or gastric cancer; by administration of an epothilone as a cytotoxic agent, especially epothilone B.

Generally, a composition of the present invention may be administered in an amount which is therapeutically effective against a proliferative disease that can be treated by administration of an epothilone, e.g. epothilone A and/or epothilone B, especially epothilone B. Such proliferative diseases include any proliferative disease as mentioned above, especially a tumour disease, the response to a therapeutically effective amount preferably manifesting itself in a diminished proliferation, e.g. diminished tumour growth or even (more preferably) tumor regression or (most preferably) tumour disappearance. The exact amount and the duration of administration may depend upon the nature of the epothilone, e.g. epothilone A, epothilone B or a mixture of both, the particular type of malignantly proliferating cells characteristic of the particular tumour, the seriousness of the condition, the rate of administration, as well as the patient's health and response to treatment.

Compositions of the present invention may be combined with other tumour treatments known to a skilled person, e.g. radiation, or administered as part of a combination therapy comprising at least one other chemotherapeutic agent. The administration of a combination of active agents may be simultaneous or consecutive, with either one of the active agents being administered first. The dosage of the active agents of a combination treatment may depend on effectiveness and site of action of each active agent as well as synergistic effects between the agents used for combination therapy.

Other chemotherapeutic agents may include especially any chemotherapeutic agent that is or can be used in the treatment of tumor diseases, such as chemotherapeutics derived from the following classes:
(A) alkylating agents, preferably cross-linking chemotherapeutics, preferably bis-alkylating agents,
(B) antitumour antibiotics, preferably doxorubicin (Adriamycin®, Rubex®);
(C) antimetabolites;
(D) plant alkaloids;
(E) hormonal agents and antagonists;
(F) biological response modifiers, preferably lymphokines or interferons;
(G) inhibitors of protein tyrosine kinases and/or serine/threonine kinases;
(H) antisense oligonucleotides or oligonucleotide derivatives; or
(I) miscellaneous agents or agents with other or unknown mechanism of action, preferably of the Taxane class, especially Taxotere® or most especially paclitaxel (Taxol®).

Compositions of the present invention may, therefore, be useful as single anti-cancer compositions or as part of a combination regimen for the treatment of various tumours.

The utility of all compositions of the present invention may be observed in standard clinical trials in, for example, known indications of epothilone dosages giving equivalent blood levels of epothilone; for example using dosages in the range of about 0.1 to 6 mg/m² or higher, e.g. to 10 mg/m², of epothilone for weekly treatment and about 0.3 to 18 mg/m² or higher, e.g. to 30 mg/m², of epothilone for three-weekly treatment for a 75 kilogram mammal, e.g. an adult human of 1.73 m², and in standard animal models. For example, the anti-tumor effect of single dose regimens are investigated in a model of human ovarian cancer SKOV3 as well as a U373 glioma model.

The increased bioavailability of an epothilone administered in the form of a composition of the present invention, may be observed in standard animal tests and in clinical trials, e.g. as described above. Naturally, the exact amounts of epothilone, e.g. epothilone-liposome, and of the composition to be administered may depend on a number of factors, e.g. the condition to be treated, the exact epothilone, the desired duration of treatment and the rate of administration of epothilone. For example, the amount of epothilone required and the administration rate thereof may be determined on the basis of known *in vivo* and *in vitro* techniques, for example as described above, determining how long a particular epothilone concentration in the blood plasma remains at an acceptable level for a therapeutic effect.

Compositions of the present invention may be conveniently administered, e.g. intravenously, in a dosage of from about 0.1 to 100 mg/m², e.g. 0.2 to 100 mg/m² epothilone A and from about 0.1 to 50 mg/m², e.g. 0.1 to 20 mg/m² of epothilone B.

Preferably the concentration and dosage strength may be such to achieve an effective dose level of about 0.1 to 15 mg/day or higher e.g. to 20 mg/day, more preferably 0.1 to 10 mg/day, more preferably 0.1 to 8 mg/day. The dose received by intravenous administration and the blood concentration may be determined accurately on the basis of known *in vivo* and *in vitro* techniques.

In yet another aspect the invention provides a method of administering an epothilone to a subject in need of epothilone treatment which comprises administering parenterally a composition of the present invention to a subject in need of such treatment.

In yet another aspect the invention provides use of an epothilone in the manufacture of a medicament in liposomal form.

The invention is illustrated by way of the following examples which are not intended to limit the scope of the present invention. All percentages are by weight/weight unless otherwise specified. Any components of the compositions of the present invention may further be described in Fiedler, H. P. "Lexikon der Hilfsstoffe für Pharmazie, Kosmetik und angrenzende Gebiete", Editio Cantor, D-7960 Aulendorf, 4th revised and expanded edition (1996), the contents of which are hereby incorporated by reference.

### EXAMPLES

### Example 1: Composition of a liposomal composition comprising Epothilone B:

| **Ingredient** | **Content** |
|---|---|
| Epothilone B | 1.00 mg |
| Phosphatidylcholine (Lipoid S 100) | 16.25 mg |
| Cholesterol | 3.75 mg |
| MPEG-DSPE | 5.00 mg |
| Lactose | 80.00 mg |
| Citric acid | 4.20 mg |
| Tartaric acid | 6.00 mg |
| NaOH | 5.44 mg |
| Water up to | 1 ml |

The liposomal compositions were prepared according to standard procedures. A mixture of lipids and epothilone B were dissolved in ethanol and the solution was dried as a thin film by rotation under reduced pressure. The resultant lipid film was hydrated by addition of the aqueous phase and the particle size of the epothilone-liposomes was adjusted using standard methods.

### Example 2: liposomal composition comprising Epothilone B:

| **Component** | **Amount** |
|---|---|
| Epothilone B | 1.00 mg |
| Phosphatidylcholine (Lipoid S 100) | 19.80 mg |
| Cholesterol | 3.75 mg |
| DSPC | 1.45 mg |
| Lactose | 80.00 mg |
| Citric acid | 4.20 mg |
| Tartaric acid | 6.00 mg |
| NaOH | 5.44 mg |
| Water up to | 1 ml |

| | |
|---|---|
| DSPC = distearyl phosphatidylcholine | |

The composition is prepared analogously to that in Example 1.

### Example 3: liposomal composition comprising Epothilone B:

| **Component** | **Amount** |
|---|---|
| Epothilone B | 1.00 mg |
| Phosphatidylcholine | - |
| Cholesterol | - |
| POPC | 17.50 mg |
| POPG, Na | 7.50 mg |
| Lactose | 80.00 mg |
| Citric acid | 4.20 mg |
| Tartaric acid | 6.00 mg |
| NaOH | 5.44 mg |
| Water up to | 1 ml |

| | |
|---|---|
| POPC = 1-palmitoyl-2-oleyl-sn-glycero-3-phosphocholin. | |
| POPG = 1-palmitoyl-2-oleyl-sn-glycero-3-phosphoglycerol. | |

The composition is prepared analogously to that in Example 1.

### Example 4:

The effectiveness of the composition of Example 1 is seen upon administration of the composition against DU145 tumors in mice (prostrate carcinoma). 4 mg/kg of liposomal epothilone B administered i.v. on days 13 and 28 results in survival of 6 mice out of 6 after 48 days. In comparison, 4 mg/kg of an epothilone B solution administered i.v. on days 13 and 35 results in survival of 4 mice out of 6 on day 41 and no survival on day 44.

This invention provides epothilone compositions which may exhibit an improvement in tolerability over free epothilone.

## Claims

1. A liposomal composition comprising an epothilone.

2. A pharmaceutical composition comprising an epothilone-liposome.

3. A composition according to claims 1 or 2 comprising a polyethyleneglycol-derivatized phospholipid.

4. A composition according to any of the preceding claims in dry form.

5. A composition according to any of claims 1 to 3 comprising a pharmaceutically acceptable solvent.

6. A reconstituted form of a composition according to claim 4 comprising a pharmaceutically acceptable solvent.

## Patentansprüche

1. Liposomale Zusammensetzung, umfassend ein Epothilon.

2. Pharmazeutische Zusammensetzung, umfassend ein Epothilonliposom.

3. Zusammensetzung gemäß Anspruch 1 oder 2, umfassend ein durch Polyethylenglykol derivatisiertes Phospholipid.

4. Zusammensetzung nach einem der vorstehenden Ansprüche in trockener Form.

5. Zusammensetzung gemäß einem der Ansprüche 1 bis 3, umfassend ein pharmazeutisch annehmbares Lösemittel.

6. Wiederhergestellte Form einer Zusammensetzung gemäß Anspruch 4, umfassend ein pharmazeutisch annehmbares Lösemittel.

## Revendications

1. Composition liposomale comprenant une épothilone.

2. Composition pharmaceutique comprenant un épothilone-liposome.

3. Composition selon l'une des revendications 1 ou 2 comprenant un phospholipide substitué par polyéthylèneglycol.

4. Composition selon l'une quelconque des revendications précédentes sous forme sèche.

5. Composition selon l'une quelconque des revendications 1 à 3 comprenant un solvant pharmaceutiquement acceptable.

6. Forme reconstituée d'une composition selon la revendication 4 comprenant un solvant pharmaceutiquement acceptable.
